# EUROPEAN PATENT SPECIFICATION

(11) **EP 1 890 713 B1**
(45) Date of publication and mention of the grant of the patent: **30.03.2011**
(21) Application number: 05774219.9
(22) Date of filing: 03.08.2005
(51) Int. Cl.: A61K 35/32

(54) **OSTEOBLAST COMPOSITION OF SEMI-SOLIDIFIED MIXED FIBRIN FOR BONE FRACTURE AGGLUTINATION AND ITS MANUFACTURING METHOD**
OSTEOBLASTEN-ZUSAMMENSETZUNG AUS HALBVERFESTIGTEM GEMISCHTEM FIBRIN ZUR AGGLUTINATION VON KNOCHENFRAKTUREN UND HERSTELLUNGSVERFAHREN DAFÜR
COMPOSITION OSTEOBLASTIQUE A BASE DE FIBRINE MELANGEE SEMI-SOLIDIFIEE UTILISEE POUR PRODUIRE UNE AGGLUTINATION EN CAS DE FRACTURE OSSEUSE ET SON PROCEDE DE PRODUCTION

(30) Priority: 13.06.2005 KR 20050050450
(43) Date of publication of application: 27.02.2008
(73) Proprietor: Sewon Cellontech Co., Ltd., Youngdeungpo-Gu, Seoul 150-712 (KR)
(72) Inventor: PARK, Hyun-Shin, Sinjeong 6(yuk)-dong, Yangcheon-gu, Seoul 158-774 (KR); JANG, Jae-Deog, Junggye 2(i)-dong, Nowon-gu, Seoul 139-784 (KR); CHANG, Cheong-Ho, Banpo-dong, Seocho-gu, Seoul 137-040 (KR); JUNG, Soo-Jin, Gyomun-dong 826, Guri-si, Gyeonggi-do 471-020 (KR); LEE, Sae-Bom, Gwangjin-gu, Seoul 143-769 (KR); KO, Chang-Kwon, Nowon-gu, Seoul 139-921 (KR)
(74) Representative: Hollatz, Christian
(86) International application number: PCT/KR2005/002531
(87) International publication number: WO 2006/135126

(56) References cited:
- EP-A- 1 535 633
- EP-A2- 1 259 196
- WO-A-01/85225
- WO-A-03/007873
- WO-A-2004/110512
- DE-A1- 19 949 290
- KR-A- 2003 081 821
- US-A- 5 972 703
- KNESER U. ET AL.: 'Fibrin gel-immobilized primary osteoblasts in calcium phosphate bone cement: in vivo evaluation with regard to application as injectable biological bone substitute' CELLS TISSUES ORGANS vol. 179, no. 4, 2005, pages 158 - 169, XP008074203

## Description

### [Technical Field]

The present invention relates to a semi-solid osteoblast composition containing fibrin for bone union and a method for preparing the same. More specifically, the present invention relates to a semi-solid osteoblast composition containing fibrin for bone union, which provides a therapeutic agent containing a mixture of osteoblasts and fibrin, capable of being injected into an affected part for bone union in treatment of severe bone fracture; and a method for preparing the same.

### [Background Art]

Generally, a simple bone fracture may be sufficiently healed by casting the bone fracture for several weeks, but a severe bone fracture or bone defect requires bone grafting.

A method of bone grafting, preferred by most physicians, is autografting which involves collecting bone from a part of the patient body, particularly iliac crest, followed by grafting to the affected part of the patient. This method can provide excellent results due to grafting of the patient's own bone. However, such autografting is disadvantageously a surgical technique accompanying very severe pain, and presents a variety of potential problems associated with probable occurrence of tenderness and sensory loss in a bone-harvested region, necessity of additional surgical operation for bone grafting, and thus a long period of time for hospitalization and recovery leading to increased medical fees. In addition, there is no sufficient extra bone for grafting in the body and thus autografting suffers from a great deal of difficulty to secure donor parts for providing bone for bone implantation. In the case of a spinal surgery necessitating a great deal of bone grafting, incidence of complications caused by bone autografting is reported to be as high as about 23%.

Allografting, which has been used to alleviate these shortcomings exhibited by autografting, primarily obtains bones from corpses and is advantageous due to no need for an additional secondary surgery for bone grafting, but suffers from fatal disadvantages such as weakening of bone strength during a sterilization process, occurrence of graft rejection, and probability of infection with contagious diseases such as hepatitis and AIDS. In addition, allografting has disadvantages such as poor bone-forming effects, immune reaction, bone absorption and refracture of the corresponding bones, thus limiting application thereof to a very narrow range. Consequently, use of this surgical technique has been decreased from late 1993 since Food & Drug Administration has tightened regulations on bone tissue banks against infection.

Meanwhile, bone marrow infection is a technique based on assertion, proposed by Huggins (1931), Friedenstein (1973), and Ashton (1980), wherein osteoprogenitor cells from bone marrow induce and facilitate bone formation. Bone marrow injection is generally carried out alone for healing bone fractures, but is also carried out in combination with bone grafting. Unlike other bone grafting techniques, this bone marrow injection does not involve surgical skin incision for donor parts and thus is advantageous due to no donor site morbidity and no complications or adverse side effects.

Thereafter, even though some excellent results are published through great deal of clinical application cases, bone marrow injection is disadvantageous due to its unsound theoretical basis, by the reasons that considerable portions of results obtained are not consistent therebetween, the amount of bone marrow collectable from one site is limited and further, significantly limited number of osteoprogenitor cells are present in bone marrow.

### [Disclosure]

### [Technical Problem]

Therefore, the present invention has been made in view of the above problems, and it is an object of the present invention to provide a semi-solid osteoblast composition containing fibrin for bone union and a method for preparing the same, having no clinical graft rejection via injection of an osteoblasts and fibrin mixture into a site where bone union is sought, and capable of achieving effective and rapid bone union via injection of a composition which was shaped to a certain extent, in order to alleviate problems associated with probability of bone tissue formation in unwanted regions resulting from escape of injected osteoblasts from the site for bone union and then propagation thereof to other sites via the blood stream, which could be caused by injection of an osteoblast suspension alone.

It is another object of the present invention to provide a semi-solid osteoblast composition containing fibrin for bone union and a method for preparing the same, which are capable of performing a surgical operation with minimal incision when osteoblasts and fibrin are injected into a site where bone union is sought, and which are capable of achieving bone union via simple and effective injection of a mixed composition of osteoblasts and fibrin without incision of the corresponding damaged regions upon utilizing radiation.

### [Technical Solution]

In accordance with an aspect of the present invention, the above and other objects can be accomplished by the provision of a method for preparing a semi-solid osteoblast composition containing fibrin for bone union, comprising:
isolating osteoblasts from a bone tissue and culturing/proliferating the isolated osteoblasts in DMEM (Dulbecco's Modified Eagle's Medium) or a - MEM (Minimum Essential Medium, Alpha Modification) to prepare an osteoblast suspension; and
mixing the resulting osteoblast suspension with a coagulation factor to prepare an osteoblast therapeutic agent.

Herein, the coagulation factor is a mixture of thrombin and fibrinogen.

Herein, preparation steps of the therapeutic agent includes dissolving lyophilized thrombin in liquid DMEM or a -MEM and then mixing 1 to 100 IU/mL of the dissolved thrombin with the osteoblast mixed solution; and dissolving lyophilized fibrinogen in liquid DMEM or α-MEM and then mixing 20 to 100 mg/mL of the dissolved fibrinogen with the osteoblast mixed solution containing thrombin mixed therein.

In accordance with another aspect of the present invention, there is provided an osteoblast composition prepared by the above-mentioned method for preparing a semi-solid osteoblast composition containing fibrin for bone union.

### [Advantageous Effects]

In accordance with a semi-solid osteoblast composition containing fibrin for bone union of the present invention and a method for preparing the same, having construction as described above, it enables bone grafting, having no clinical graft rejection via injection of an osteoblasts and fibrin mixture into a site where bone union is sought, and capable of achieving effective and rapid bone union via injection of a composition which was shaped to a certain extent, in order to alleviate problems associated with probability of bone tissue formation in unwanted regions resulting from escape of injected osteoblasts from the site for bone union and then propagation thereof to other sites via the blood stream, which are caused by injection of an osteoblast suspension alone.

In addition, the present invention enables bone grafting which is capable of performing a surgical operation with minimal incision when osteoblasts and fibrin are injected into a site where bone union is sought, and which is capable of achieving bone union via simple and effective injection of a mixed composition of osteoblasts and fibrin without incision of the corresponding damaged regions upon utilizing radiation.

### [Description of the Drawings]

The above and other objects, features and other advantages of the present invention will be more clearly understood from the following detailed description taken in conjunction with the accompanying drawings, in which:
Fig. 1 is a view showing fibrin polymerization cascade;
Fig. 2 is a schematic diagram showing polymerization of a fibrin matrix;
Fig. 3 is a flow chart showing a process for preparing a semi-solid osteoblast composition containing fibrin for bone union in accordance with the present invention;
Fig. 4 is a photograph showing injection of a fibrin and osteoblast mixture after introduction of 15 mm-sized bone fracture into a forearm of a rabbit;
Fig. 5 is a radiograph taken 6 weeks after injection of a semi-solid osteoblast therapeutic agent containing a fibrin and osteoblast mixture following introduction of 15 mm-sized bone fracture into a forearm of a rabbit;
Fig. 6 is a photograph of a tissue section stained with Hematoxylin-Eosin, taken 6 weeks after injection of a semi-solid osteoblast therapeutic agent containing a fibrin and osteoblast mixture following introduction of 15 mm-sized bone fracture into a forearm of a rabbit; and
Fig. 7 is a photograph of a tissue section stained with Masson's Trichrome, taken 6 weeks after injection of a fibrin and osteoblast mixture following introduction of 15 mm-sized bone fracture into a forearm of a rabbit.

### [Best Mode]

Hereinafter, a semi-solid osteoblast composition containing fibrin for bone union in accordance with the present invention and a method for preparing the same will be described in more detail with reference to the accompanying drawings.

In order to overcome disadvantages exhibited by conventional therapies for treating bone-related disorders and diseases, such as implants and bone grafting, a technique of transplanting autologous osteoblasts having no adverse side effects on an affected part, via culturing of osteoblasts, as an ultimate treatment method, has been developed, in conjunction with improvement in conventional therapies.

However, for achieving earlier bone union, it is necessary to mix osteoblasts with a matrix through which more diverse lesions can be treated, and thereby it is possible to treat more bone diseases that do not share therapeutic benefits with injection of a liquid osteoblast suspension consisting of osteoblasts only.

It can be said that the semi-solid osteoblast therapeutic agent for bone union, which is a mixture of osteoblasts and fibrin, is a more advanced product of an injection method of a liquid osteoblast suspension which is based on cell therapy. The conventional injection method of a liquid osteoblast suspension, which involves transplanting osteoblasts alone, can only treat limited types of bone defects and may suffer from problems associated with probability of bone tissue formation in unwanted regions resulting from escape of injected osteoblasts from bone-forming sites and then propagation thereof to other sites via the blood stream. In contrast, the semi-solid osteoblast therapeutic agent containing fibrin in accordance with the present invention is made up of a mixture of osteoblasts and fibrin and therefore, can also more rapidly and effectively treat a broader range of bone fractures and severe bone diseases.

Fibrin glue is reported to promote neovascularization in animal models (Schlag G. Clinical Orthopedics, 1988), and is very effective to enhance applicability of methods used in grafting of implant materials for bone replacement, such as demineralized bone powder, bioactive glass, hydroxyapatite and coral granules, upon performing surgical grafting operation of such materials.

In addition, fibrin glue serves as an effective delivery vehicle of bioactivators for a bone matrix. As examples of such bioactivators, mention may be made of TGF-β-1 (Transforming growth factor-beta-1) and TGF-β-related BMP (Transforming growth factor-beta related Bone morphogenetic protein). Recently, fibrin glue has received a great deal of attention as a matrix constituent material that effectively delivers cells, in tissue-engineering applications (Huang Q. Tissue Engineering 2002). The above-mentioned characteristics of fibrin glue lead to a rapid bone union process in the present composition involving mixing osteoblasts with fibrin glue as a matrix material, followed by application for bone union.

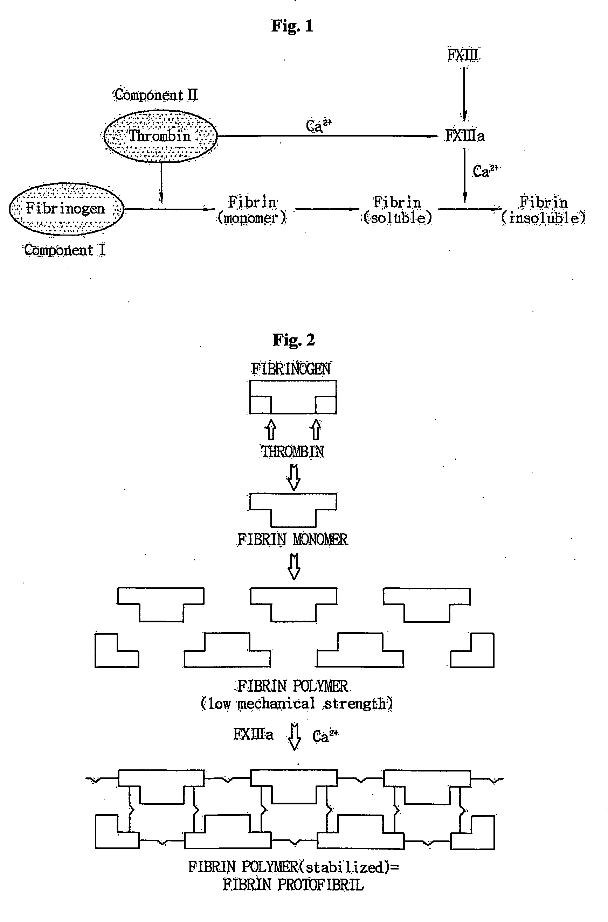

Referring to the drawings, Fig. 1 shows fibrin polymerization cascade, and Fig. 2 schematically shows polymerization of a fibrin matrix.

For healing of bone fracture, immobilization of the fracture regions should be preceded and sufficient supply of blood and application of appropriate stress to the bone fracture regions should be ensured. Bone fracture, once occurred, is usually accompanied by damage to skin, muscles, ligaments, blood vessels, nerves and joints therearound.

Upon reviewing histologically, healing processes of bone fracture involves three phases, i.e., inflammation, repair and remodeling phases, and these stages are not strictly divided but progress continuously with being overlapped therebetween.

The inflammation phase is a stage in which cells in bone matrix, blood vessels, and also soft tissues such as periostea and muscles around bone fracture regions are damaged due to fracture- causing trauma, resulting in formation of hematoma in a medullary cavity and beneath the periosteum. Inflammatory derivatives liberated from damaged necrotic cells lead to vasodilation and exudation of blood plasma, thereby causing acute edema. After the inflammation phase, the switchover to a repair phase is followed with initiation of new matrix formation.

The repair phase is initiated with organization of hematoma formed in the fractured sites. Hematoma creates a fibrin scaffold which then receives repair cells. Then, growth factors or other proteins formed by these repair cells induce cell migration and proliferation, and formation of a repair matrix, which are the first step of bone fracture restoration. Majority of cells creating bone tissues during healing of bone fracture appear in the fracture site together with granulation tissues, and form calluses, which are made up of fibrous tissues, cartilage and woven bones, around the fracture site. Mineralization of callus is initiated by serial actions of osteogenic cells. The matrix, which is synthesized by these osteogenic cells, contains a large number of regularly-spaced type I collagen fibers that provide conditions for deposition of calcium phosphate complexes in these spaces. However, this repair phase is not a stage in which bone union has been completed, and thus the resulting immature fracture calluses have weaker bone strength than normal bone tissues. Therefore, during the remodeling stage, such fracture calluses acquire the strength as exhibited in normal bone tissues.

As to the remodeling stage, woven bones within calluses are replaced by mature lamellar bones, and unnecessary and superfluous calluses are gradually absorbed. Based on the results of radioisotope examination, it can be seen that increased activity of radioisotope is maintained in the fracture site for a prolonged period of time, even after completion of bone union and recovery of functions of the fracture site to normal state, as shown on the radiograph. Therefore, the remodeling stage continues for several years after clinical union of bone fracture has occurred.

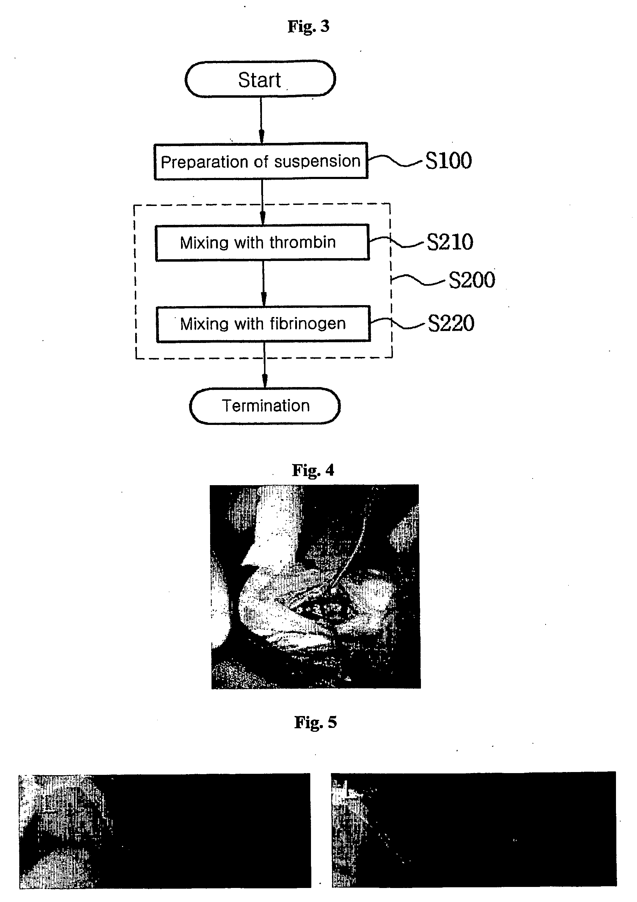

Fig. 3 shows a flow chart illustrating a process for preparing a semi-solid osteoblast composition containing fibrin for bone union in accordance with the present invention.

Referring now to Fig. 3, a method for preparing a semi-solid osteoblast composition containing fibrin for bone union in accordance with the present invention, comprises isolating osteoblasts from a bone tissue and culturing/proliferating the isolated osteoblasts in DMEM (Dulbecco's Modified Eagle's Medium) or α-MEM (Minimum Essential Medium, Alpha Modification) to prepare an osteoblast suspension (S100); and mixing the resulting osteoblast suspension with a coagulation factor to prepare an osteoblast therapeutic agent (S200).

Herein, the coagulation factor is a mixture of thrombin and fibrinogen.

Herein, preparation steps of the therapeutic agent include dissolving lyophilized thrombin in liquid DMEM or α-MEM and then mixing 1 IU/mL to 100 IU/mL of the dissolved thrombin with the osteoblast mixed solution (S210); and dissolving lyophilized fibrinogen in liquid DMEM or α-MEM and then mixing 20 to 100 mg/mL of the dissolved fibrinogen with the osteoblast mixed solution containing thrombin mixed therein (S220).

Meanwhile, a concentration of thrombin determines a polymerization time of fibrin. Therefore, the polymerization time of fibrin can be reduced within a range of 4 hours to 3 sec, depending upon the concentration of thrombin. Thrombin was applied to the concentration such that the composition of the present invention is shaped to prevent osteoblasts and coagulation factors from leaking from lesion sites when injected into a site for union of bone fracture, injection of the composition into the affected site leads to rapid formation of a matrix, and an optimal fibrin pore matrix for bone formation by osteoblasts is formed.

### [Mode for Invention]

Hereinafter, effects of a semi-solid osteoblast composition containing fibrin for bone union in accordance with an example of the present invention will be described in more detail with reference to the accompanying drawings.

Firstly, osteoblasts were isolated from the corresponding tissues, and cultured in DMEM or a-MEM for 4 weeks to proliferate sufficient numbers of osteoblasts. Then, the resulting osteoblasts were suspended in DMEM or a-MEM to prepare a suspension of osteoblasts.

After preparing a medical-grade fibrin glue set at room temperature, fibrinogen was dissolved by adding a proper quantity of liquid DMEM or α-MEM to a vial containing lyophilized fibrinogen.

In addition, thrombin was also dissolved by adding a proper quantity of liquid DMEM or α-MEM to a vial containing the lyophilized thrombin.

Then, the dissolved thrombin was added in a 1/5 volume to the liquid osteoblast suspension and mixed well.

An osteoblast suspension mixed with thrombin was mixed with an equal amount of fibrinogen to prepare 500 µl of an osteoblast composition.

30 New Zealand white rabbits, weighing about 3 kg, disregarding sex, were divided into a control group for bone grafting, and an experimental group for grafting a semi-solid osteoblast composition containing fibrin for bone union, each group consisting of 15 rabbits.

According to a Henry's approach, a longitudinal incision was made in the forearm of the rabbits, thereby exposing shaft of radius, and then 15 mm-sized bone defect was made using a saw, followed by complete removal of the periosteum from the bone defect area.

In the control group, cancellous bone was previously collected from long bone and bone grafting was carried out in the bone defect area, followed by suturing skin and subcutaneous tissues.

In the experimental group, skin and subcutaneous tissues were carefully sutured such that the bone defect area becomes a vacant space, and the osteoblast composition was injected three weeks later, as shown in Fig. 4.

After taking radiographs at a week 3, 6 and 9 from initiation of experiment, bone union was evaluated by assignment of the corresponding point(s) to proximal and distal osteotomy areas and bone defect area, respectively, depending upon a degree of bone union thereof, and the sum of the thus-gained points.

**Table 1**

| Method for determining union of bone fracture for lesions | | | |
|---|---|---|---|
| Point | Proximal osteotomy area | Distal osteotomy area | Mild portion of defect |
| 3 | Union | Union | Union |
| 2 | Moderate bridge (> 50%) | Moderate bridge (> 50%) | Moderate bridge (> 50%) |
| 1 | Mild bridge (< 50%) | Mild bridge (< 50%) | Mild bridge (< 50%) |
| 0 | Nonunion | Nonunion | Nonunion |

**Table 2**

| Results after nine weeks of bone grafting in control and experimental groups | | |
|---|---|---|
| | Control group | Experimental group |
| Excellent (8-9) | 14 | 13 |
| Good (6-7) | 1 | 2 |
| Fair (4-5) | - | - |
| Poor (2-3) | - | - |
| No effect (0-1) | -- | - |

Fig. 5 shows a radiograph taken 6 weeks after injection of a semi-solid osteoblast therapeutic agent containing a fibrin and osteoblast mixture following introduction of 15 mm-sized bone fracture into the forearm of a rabbit and it can be seen that complete union of bone fracture was achieved.

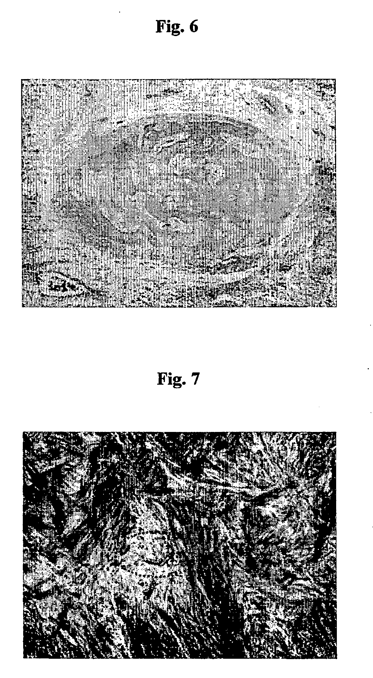

Fig. 6 shows a photograph of a tissue section stained with Hematoxylin-Eosin, taken 6 weeks after injection of a semi-solid osteoblast therapeutic agent containing a fibrin and osteoblast mixture following introduction of 15 mm-sized bone fracture into the forearm of a rabbit and there was observed a bone island formed alone.

Fig. 7 shows a photograph of a tissue section stained with Masson's Trichrome, taken 6 weeks after injection of a fibrin and osteoblast mixture following introduction of 15 mm-sized bone fracture into the forearm of a rabbit, and it can be confirmed that collagen was produced along predetermined patterns, thus representing progress of bone formation and formation of blood vessels therebetween (a red elliptic dotted line).

Thus, in accordance with the present invention, there is provided bone grafting without immunological rejection by mixing osteoblasts with fibrin and injecting the mixture into affected parts for bone union, when bone union does not progress due to severe bone fracture, and exhibiting effective and rapid bone union effects via injection of a composition which was shaped to a certain extent.

### [Industrial Applicability]

As apparent from the foregoing, in accordance with a semi-solid osteoblast composition containing fibrin for bone union of the present invention and a method for preparing the same, having formulation as described above, it enables bone fracture reunion, having no clinical graft rejection via injection of an osteoblasts and fibrin mixture into a site where bone union is sought, and capable of achieving effective and rapid bone union via injection of a composition which was shaped to a certain extent, in order to alleviate problems associated with probability of bone tissue formation in unwanted regions resulting from escape of injected osteoblasts from the site for bone union and then propagation thereof to other sites via the blood stream, which may be caused by injection of an osteoblast suspension alone.

In addition, the present invention enables bone fracture reunion which is capable of performing a surgical operation with minimal incision when osteoblasts and fibrin are injected into a site where bone union is sought, and which is capable of achieving bone union via simple and effective injection of a mixed composition of osteoblasts and fibrin without incision of the corresponding damaged regions upon utilizing radiation system such as X-ray.

Although the preferred embodiments of the present invention have been disclosed for illustrative purposes, those skilled in the art will appreciate that various modifications, additions and substitutions are possible.

## Claims

1. A fibrin containing semi-solid osteoblast composition for curing bone fracture
wherein the composition is prepared by a method comprising:
culturing/proliferating isolated osteoblasts from a bone tissue in Minimum Essential Medium Alpha Modification (alpha-MEM) to prepare an osteoblast suspension, and
mixing the resulting osteoblast suspension with a coagulation factor to prepare an osteoblast therapeutic agent, wherein said coagulation factor is a mixture of thrombin and fibrinogen,
wherein the preparation steps of the therapeutic agent include:
dissolving lyophilized thrombin in liquid alpha-MEM and then mixing 1 to 100 IU/mL of the dissolved thrombin with the osteoblast mixed solution, and
dissolving lyophilized fibrinogen in liquid alpha-MEM and then mixing 20 mg/mL to 100 mg/mL of the dissolved fibrinogen with the osteoblast mixed solution containing thrombin mixed therein.

## Patentansprüche

1. Fibrinhaltige halbfeste Osteoblastenzusammensetzung zur Heilung von Knochenbruch, wobei die Zusammensetzung durch ein Verfahren hergestellt ist, umfassend:
Kultivieren/Proliferieren von isolierten Osteoblasten aus einem Knochengewebe in Minimum Essential Medium Alpha Modification (alpha-MEM), um eine Osteoblastensuspension herzustellen, und
Mischen der resultierenden Osteoblastensuspension mit einem Gerinnungsfaktor, um ein therapeutisches Osteoblastenmittel herzustellen, wobei der Gerinnungsfaktor eine Mischung aus Thrombin und Fibrinogen darstellt, wobei die Herstellungsschritte des therapeutischen Mittels umfassen:
Auflösen von lyophilisiertem Thrombin in flüssigem alpha-MEM und anschließendem Mischen von 1 bis 100 IU/mL des aufgelösten Thrombins mit der gemischten Osteoblastenlösung, und
Auflösen von lyophilisiertem Fibrinogen in flüssigem alpha-MEM und anschließendem Mischen von 20 mg/mL bis 100 mg/mL des aufgelösten Fibrinogens mit der gemischten Osteoblastenlösung, enthaltend darin gemischtes Thrombin.

## Revendications

1. Composition semi-solide d'ostéoblastes, contenant de la fibrine, conçue pour soigner une fracture osseuse, laquelle composition a été préparée selon un procédé comprenant les étapes suivantes :
- cultiver et faire proliférer des ostéoblastes isolés à partir d'un tissu osseux, dans de la variante alpha du milieu essentiel minimal (MEM-α), afin de préparer un suspension d'ostéoblastes,
- et mélanger avec un facteur de coagulation la suspension d'ostéoblastes ainsi obtenue, afin de préparer un agent thérapeutique à base d'ostéoblastes, ledit facteur de coagulation étant un mélange de thrombine et de fibrinogène ;
étant entendu que les étapes de préparation de cet agent thérapeutique incluent les suivantes :
- dissoudre de la thrombine lyophilisée dans du milieu MEM-α liquide et mélanger ensuite cette thrombine dissoute, à raison de 1 à 100 UI/mL, avec la solution mêlée d'ostéoblastes,
- puis dissoudre du fibrinogène lyophilisé dans du milieu MEM-α liquide et mélanger ensuite ce fibrinogène dissous, à raison de 20 à 100 mg/mL, avec la solution mêlée d'ostéoblastes contenant de la thrombine mélangée dedans.
